# EUROPEAN PATENT APPLICATION

(11) **EP 1 884 508 A1**
(43) Date of publication of application: **06.02.2008**
(21) Application number: 06015770.8
(22) Date of filing: 28.07.2006
(51) Int. Cl.: C07C 37/50, C07C 67/343, C07C 69/738, C07C 49/84, C07C 43/23, C07C 39/19

(54) **Process for the preparation of polyhydroxylated stilbenes via claisen condensation**

(71) Applicant: Portela & Ca., S.A., 4745-457 S. Mamede do Coronado (PT)
(72) Inventor: Wiffen, Jonathan, William, Craigavon, Co Armagh BT67 0UB (GB); McCague, Raymond, Coton, Cambridge CB23 7PX (GB)
(74) Representative: Bublak, Wolfgang

(57) **Abstract**

The invention realates to a Process for the preparation of resveratrol, comprising the steps of
a) reaction of a compound according to formula I 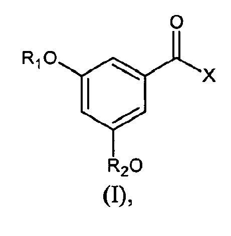 with a compound according to formula II 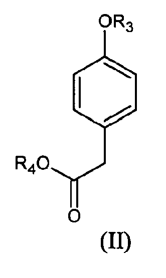 to form a compound according to formula III 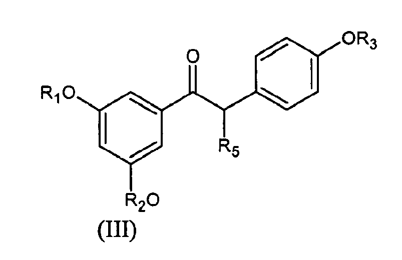 b) conversion of the compound according to formula III to a compound according to formula IV 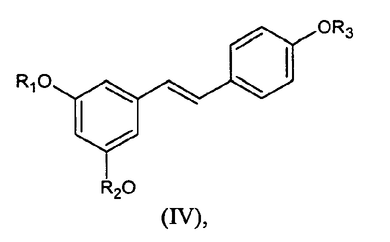 and, if necessary,
c) deprotection of the compound according to formula IV.

## Description

Polyhydroxylated trans-stilbenes have been found to exhibit interesting biological activities. Trans-resveratrol which in IUPAC nomenclature corresponds to (*E*)-1-(3,5-dihydroxyphenyl)-2-(4-hydroxyphenyl)ethene has been found to raise the level of high-density lipoproteins (HDL) and lower the level of low-density lipoproteins (LDL) in human beings thereby reducing the risk of clogging arteries and consequent myocardial infarctions (Toppo, F. U.S. Pat. No. 6,048,903). Many 5-alkenyl resorcinols show antileukemic activity (Alonso, E; Ramon, D. J; Yus, M. J. Org. Chem. 1997, 62, 417-421; Thakkar, K; Geahlen, R. L.; Cushman, M. J. Med. Chem. 1993, 36(20), 2950-2955) and anti-tumor properties (He, K.; Zheng, Q. Y.; Zheng, B. L.; Kim, C. H. WO 2000/037021). Plant material containing trans-resveratrol has been used as herbal medicine for the treatment of hyperlupemia and liver diseases in China and Japan for many centuries (Kimura, K. M. et al. Shoyqakugaku Zasshi 1987, 83, 35-58). Further investigations led to identification of many important biological functions including inhibition of lypooxygenase activity (Kimma, Y. et al. Biochem. Biophys. Acta. 1985, 834, 275), inhibition of anaphylactoid (Ragazy et al. Pharmacol. Commun. 1988, 79, 20) and protection of lipoproteins against oxidative and free radical damage (Frankel E. N. et al. Lancet 1979, 1, 1017).

Despite the vital biological activities of the polyhydroxylated trans-stilbenes, there have been surprisingly few methods reported for the synthesis of these compounds which mostly employ a Wittig reaction in the synthesis of resveratrol. U.S. Pat. No. 6,048,903 describes the synthesis of *E*-resveratrol by the Wittig reaction of 3,5-dimethoxybenzyltriphenyl phosphonium salt with p-anisaldehyde in the presence of n-butyl lithium. In this method the mixture of Z- and E-olefins so obtained is demethylated with boron tribromide to get very low yield of the product. This method suffers from the low quality and use of expensive and hazardous reagents. WO 00/21368 describes the condensation of phosphonate esters with aromatic aldehydes followed by demethylation using pyridine hydrochloride. In this method also the yields are low & the process is not commercially attractive.
Drewes, S. E.; Fletcher, I. P J. Chem. Soc. Perkin Trans. I 1974, 961-962 & Bajaj, R.; Gill, M. T.; McLaughlin, J. L. Rev. Latinoamer Quim. 1987, 18, 79-80 reported the synthesis of analogs of *E*-resveratrol wherein a mixture of *E*- *& Z-*isomer is obtained. Cunningham, J.; Haslam, E.; Haworth, R. D. J. Chem. Soc. 1963, 2875-2883 disclosed a different route, which comprises reaction of 3,5-dihydroxyphenylacetate and 3,4-dihydroxybenzaldehyde in acetic anhydride, decarboxylation of the ensuing 3,3',4,5'-tetraacetoxystilbene-[alpha]-carboxylic acid with copper and quinoline at high temperature, followed by hydrolysis of the resulting piceatannol tetraacetate with sodium hydroxide. The reported yields in these methods are moderate to low. Ali, M. A.; Kondo, K.; Tsuda, Y. Chem. Pharm. Bull. 1992, 40(5), 1130-1136 described the Wittig reaction, wherein the undesired Z-isomers are reported to form to the extent of 52% along with the desired E-isomers (48%) when potassium tert-butoxide is employed. Cushman, M.; Nagarathnam, D.; Gopal, D. et al. J. Med. Chem. 1992, 35 (12), 2293-2306; Chen, Yi-Ping; Lei, Tong-Kang. Zhongguo Yiyao Gongye Zazhi 2000, 31(7), 334-336 disclosed the formation of Z-isomers to the extent of 45% (E: 55%) when sodium hydride is used. These processes are commercially and technically difficult to be implemented. In addition, the main problems in the synthesis of these polyhydroxylated-1,2-diphenylethenes are caused by the instability of this polyphenolic stilbene due to facile oxidation resulting in the formation of unstable radicals and quinones.

Accordingly, it is the object of the present invention to develop an industrially viable way of producing resveratrol characterized by the use of cost-efficient, relatively low-toxic, non-hazardous starting materials and avoiding formation of undesired side products. In addition, it is a further object of the invention to develop viable and cost-efficient ways of predominantly forming the desired transisomer.

The present invention relates to processes and intermediates for the industrially viable preparation of resveratrol characterized by the use of cost-efficient, relatively low-toxic, non-hazardous starting materials and avoiding the formation of undesired side products during the reaction sequence. The process for the preparation of resveratrol comprises the steps of
a) reaction of a compound according to formula I 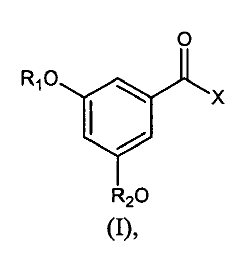 with a compound according to formula II 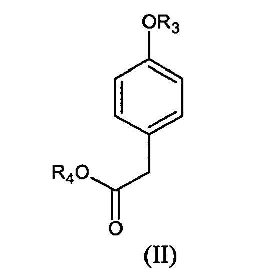 to form a compound according to formula III 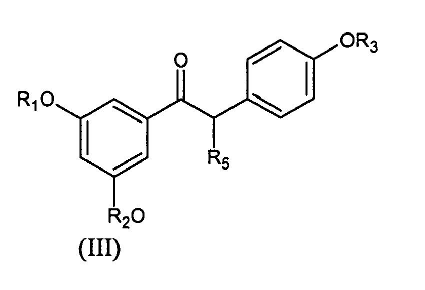
b) conversion of the compound according to formula III to a compound according to formula IV 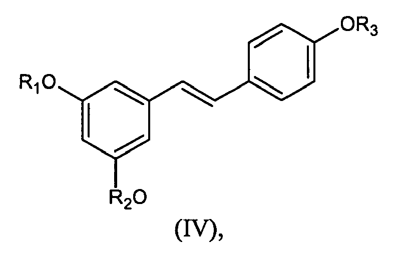 and, if necessary,
c) deprotection of the compound according to formula IV to resveratrol,
   wherein R₁, R₂, and R₃ independently from each other represent hydrogen, a (C₁-C₄)alkyl group such as methyl, ethyl, n-propyl, iso-propyl, n-butyl, iso-butyl, sec-butyl, tert-butyl; (C₁-C₄)alkoxy(C₁-C₄)alkyl group such as methoxymethyl, methoxyethyl, methoxypropyl, ethoxymethyl, ethoxyethyl, ethoxypropyl; (C₁-C₄)alkoxy(C₁-C₄)alkoxy(C₁-C₄)alkyl group such as methoxyethoxymethyl, methoxyethoxyethyl; allyl, vinyl, silyl, formyl, acyl group such as acetyl, propanoyl, butanoyl or benzoyl; aryl(C₁-C₄)alkyl or substituted aryl(C₁-C₄)alkyl group such as benzyl, phenethyl, diphenylmethyl, p-methoxybenzyl, p-nitrobenzyl, p-methylbenzyl or o-chlorobenzyl; R₄ represents a (C1-C₁₈)alkyl group such as methyl, ethyl, n-propyl, isopropyl, tert-butyl, decyl, optionally with additional substitution such as methoxyethyl, a (C₁-C₁₀)aryl group such as phenyl optionally with additional substituents on the aromatic residue such as 4-methylphenyl, 4-methoxyphenyl, or an aryl(C₁-C₄)alkyl group such as benzyl or phenethyl; R₅ represents hydrogen or a residue COOR₄; and X is a leaving group.

Most preferably, in formula I, R₁ and R₂ represent methyl.

Most preferably, in formula II, R₃ represents methyl, and R₄ represents, methyl, ethyl, n-propyl or isopropyl.

Preferred examples of suitable leaving groups X in formula I include fluoride, chloride, bromide, iodide, methoxy, ethoxy, n-propoxy, isopropoxy, n-butanoxy, sec-butoxy, tert-butoxy, phenoxy, 4-chlorophenoxy, 4-bromophenoxy, 4-nitrophenoxy, imidazolyl, acetoxy, trifluorocarboxy, tosylate and mesylate.

The reaction step a) includes a Claisen-type reaction or acylation of a compound according to formula I with a compound according to formula II to form a compound according to formula III.

In a preferred embodiment the Claisen-type reaction or acylation of a compound according to formula I with a compound according to formula II is conducted in the presence of a suitable base.

In an alternative preferred embodiment, prior to the Claisen-type reaction the compound of formula II is converted to its corresponding enolate which may optionally be silylated. The enolate is formed by deprotonation with a suitable base. The preferred silylating agent for silylating the enolate is trimethylsilyl chloride. In this case, the above described use of a base in reaction step a) is not necessary. If a silylated enolate is used an activating compound such as a Lewis acid or trimethylsilyltriflate may also be employed, as is well known in the art.

Suitable bases for deprotonating a compound according to formula II prior or in the course of the Claisen-type reaction are such bases which are strong enough to convert (at least part of) the compound according to formula II into its corresponding enolate. Preferred examples include, sodium hydride, potassium hydride, lithium diisopropylamide (LDA), lithium hexamethyldisilylamide (LHMDSA), butyl lithium, methyl lithium, potassium butoxide, alkaline alkoxides and earth alkaline alkoxides, such as sodium ethoxide, sodium methoxide, magnesium ethoxide; amidines, guanidines, tetramethylammoniumhydroxide, tetrabutylammoniumhydroxide, combinations of alkaline hydroxides, such as sodium hydroxide and potassium hydroxide, with phase transfer catalysts, and mixtures thereof.

The choice of a solvent for carrying out the reaction step a) is not particularly limited. The exact choice will *inter alia* depend on the nature and solubility of the base employed. Suitable solvents include tetrahydrofuran, dioxane, dimethoxyethane, toluene, xylenes, acetonitrile, dimethysulfoxide, dimethylformamide, dimethylacetamide, and N-methylpyrrolidone. Preferred solvents for carrying out the reaction step a) include tetrahydrofuran and dimethoxyethane.

The reaction conditions in reaction step a) are also not particularly limited and primarily depend on the choice of the leaving group X, the steric and electronic effect of the protective groups R₁ to R₄ and the type of solvent employed. Suitable temperature ranges include -70 to 100 °C, preferably -20 to 80 °C, most preferably 45 to 75 °C. Suitable reaction times range from 1 h to 48 h, preferably 2 h to 24 h, and most preferably 3 h to 18 h.

In a preferred embodiment, the Claisen-type reaction or acylation of a compound according to formula I with a compound according to formula II yields a compound of formula III in which R₅ represents COOR₄. The carboxylic ester moiety COOR₄ is hydrolysed and decarboxylated in a subsequent step. Preferably, both hydrolysis and decarboxylation are conducted in the same step under either acidic or alkaline conditions.

Preferred solvents for the hydrolysis/decarboxylation are alcohols such as methanol, ethanol, propanol, isopropanol, water and mixtures thereof. It is also possible to use the crude reaction mixture obtained in the above step of reacting as a compound according to formula II with a compound according to formula III. In such a case, it is preferred to add one of the preferred solvents for the hydrolysis/decarboxylation a second solvent to further facilitate the reaction. Suitable mixing ratios of these solvents are readily determined by the person skilled in the art.

The reaction conditions for the hydrolysis/decarboxylation are also not particularly limited and primarily depend on the choice of the protective groups R₁ to R₄ and the type of solvent employed. Suitable temperature ranges include room temperature to the boiling point of the particular solvent, preferably 40 to 110°C, more preferably 60 to 90 °C and most preferably 70 to 80 °C.

In an alternative preferred embodiment, the Claisen-type reaction or acylation of a compound according to formula I with a compound according to formula II yields a compound of formula III wherein R₅ represents H, i.e. the carboxylic ester moiety originating from the compound according to formula II is lost *in situ* under the reaction conditions of the Claisen-type reaction or acylation.

In still an alternative preferred embodiment, the groups R₁ to R₄ are changed prior to conducting the reaction steps in order to facilitate the reactions in reaction step b). In particular, it is envisaged to deprotect or transesterify the groups R₁ to R₄ or to introduce protective groups in cases where groups R₁ to R₄ represent hydroxyl groups.

The reaction step b) includes the conversion of the compound according to the formula III to the hydroxyl compound according to the formula IV.

In a preferred embodiment, the conversion of the compound according to the formula III to the compound according to the formula IV is conducted by reducing the carbonyl function and dehydrating the resulting hydroxyl compound of formula V: 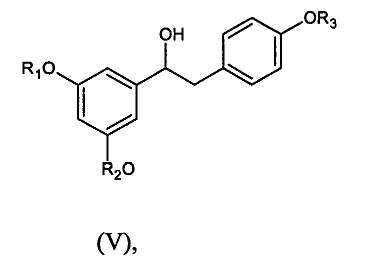 wherein R₁, R₂ and R₃ are defined as in formulas I and II.

Suitable reducing agents and reaction conditions for preparing a compound of formula V are known in the art. In a preferred embodiment, the reduction is conducted by using an ionic hydride reducing agent such as sodium borohydride or by hydrogenation such as with a Pd/C catalyst. When using sodium borohydride alcoholic solvents, such as methanol, ethanol and isopropanol, and water or mixtures thereof are preferred. When using Pd/C with hydrogen ethyl acetate and alcoholic solvents, such as methanol, ethanol and isopropanol, are preferred solvents. Suitable reaction temperatures range from 0 to 70 °C, preferably 20 to 60 °C, and most preferably 30 to 50 °C. Suitable reaction times range from 0.5 to 60 h, preferably 1 to 5 h, and most preferably 1.5 to 3 h.

Processes for the dehydration of the hydroxyl compound of formula V are known in the art. In a preferred embodiment, the hydroxyl compound of formula V is heated in an inert solvent, such as toluene, in the presence of catalytic amounts of iodine. Suitable catalytic amounts of iodine range from 3 to 50 mol%, preferably 4 to 30 mol%, more preferably 5 to 20 mol%, of the hydroxyl compound.

In an alternative preferred embodiment, the hydroxyl group of formula V is converted into a leaving group and subsequently or concomitantly the double bond is formed by elimination which is effected by the addition of a base. Preferably, the hydroxyl group is converted into a mesyl, tosyl, bromo, triflate, acetate or chloride moiety. Suitable bases for the elimination reaction which yields the double bond are known in the art. Preferably, amine bases, such as trimethylamine and triethylamine, are used. Suitable solvent systems are known in the art and preferably toluene or an ether solvent are used. Suitable reaction temperatures range from 0 to 100 °C, preferably 20 to 90 °C, and most preferably 30 to 80 °C. Suitable reaction times range from 0.5 to 5 h, preferably 1 to 4 h, and most preferably 2 to 3 h.

Most preferably, the hydroxyl compound is treated with 1 to 1.5 equivalents, preferably 1.1 to 1.4 equivalents, most preferably with 1.2 to 1.3 equivalents, mesyl chloride, and an excess, preferably 1,5 to 10 equivalents, preferably 2 to 8 equivalents, more preferably 3 to 6 equivalents, of triethylamine in toluene at 60 to 100°C, preferably 70 to 90°C, more preferably 70 to 80°C, for 3 to 8 h, preferably 4 to 7 h, more preferably 5 to 6 h. In the course of the reaction a favourable E- to Z-isomer ratio is obtained.

The optional reaction step c) relates to the deprotection of the compound according to formula IV to resveratrol. Suitable reaction conditions for the removal of protecting groups are known in the art. In a preferred embodiment, in which in formula IV R₁, R₂ and R₃ represent methyl, the protective groups are removed by treatment with BBr₃ in an inert solvent, preferably toluene, at -20 °C to 30 °C, preferably -10 to 10 °C, and most preferably at 0 to 5 °C, for 0.5 to 3 h, preferably 1 to 2 h.

### Examples

### Example 1: Preparation of 3-(3,5-dimethoxyphenyl)-2-(4-methoxyphenyl)-3-oxo-propionic acid methyl ester

A solution of methyl 4-methoxyphenyl acetate (10g, 0.055mol) in dry THF (40ml) was added dropwise over 6h to a suspension of methyl 3,5-dimethoxybenzoate (14.2g, 0.072mol) and NaH (6g, 60% suspension in oil prewashed with hexane) at 65°C under N₂. The reaction mixture was then allowed to stir at 65°C overnight. Reaction progress was monitored by TLC and ¹H NMR analyses. After complete reaction, the mixture was cooled to room temperature before concentration to half the original volume under reduced pressure. The mixture was quenched by careful addition to ice/1N HCl (150ml) followed by extraction with ethyl acetate (3 x 50ml). The combined organic extracts were washed with 10% aq. K₂CO₃ (100ml) followed by water (100ml), then dried (Na₂SO₄), filtered and concentrated yielding a viscous brown oil (18.3g, 96% crude yield). This material was used in the subsequent step without purification.

### Example 2: Preparation of 1-(3,5-dimethoxyphenyl)-2-(4-methoxyphenyl)-ethanone

The crude product of Example 1 (18.3g, 0.53mol) was dissolved in a mixture of ethanol (100ml) and 15% aq. HCl (50ml). The mixture was heated at reflux for 4h before cooling to room temperature and dilution with ethyl acetate (200ml) and water (200ml). The organic portion was then washed with 10% aq. K₂CO₃ (150ml) followed by water (100ml) before drying (Na₂SO₄), filtration and concentration in vacuo. This yielded a viscous brown oil that was recrystallised from hot isopropanol (50ml). The resultant white solid was collected by vacuum filtration and air-dried to yield 9.1g (60%) of product.

### Example 3: Preparation of 1-(3,5-dimethoxyphenyl)-2-(4-methoxyphenyl)ethanol

To a suspension of the product of Example 2 (5g, 0.017mol) in ethanol (40ml) was added NaBH₄ (0.73g, 1.1eq.) portionwise over 2 mins. The reaction mixture was then heated to 50°C and monitored by thin layer chromatography (dichloromethane eluent) until completion (ca. 1h). The reaction mixture was then concentrated to one-third of the original volume before dilution with ethyl acetate (75ml) and water (50ml). The organic portion was washed further with brine (2 x 50ml) before drying (Na₂SO₄), filtration and concentration to yield an off-white solid (5g, 99%).

### Example 4: Preparation of 3,5,4'-trimethoxystilbene

To a solution of the product of Example 3 (1.8g, 6.25mmol) in dry toluene containing Et₃N (8.8ml, 0.55mol) at 0°C was added mesyl chloride (0.58ml, 7.5mmol) dropwise. The reaction was allowed to stir at this temperature for 0.5h before heating to 80°C. The progress of the reaction was monitored by thin layer chromatography until completion (ca. 4-8h). The reaction mixture was then diluted with ethyl acetate (40ml) and 1N HCI (30ml). The organic portion was then washed further with 1N HCl (30ml) and brine (30ml) before drying (Na₂SO₄), filtration and concentration. This yielded a brown oil that was purified by flash chromatography (dichloromethane:hexane 1:1) affording a viscous colourless oil (1.2g, 71%) that solidified on standing yielding a white solid.

### Example 5: E-3,5,4'-trihydroxystilbene (Resveratrol)

To a solution of the product of Example 4 (0.5g, 1.85mmol) in dry toluene (5ml) at 0°C under N₂ was added BBr₃ (0.58ml, 5.55mmol) dropwise over 2 mins. The reaction mixture was then allowed to stir at 0°C for 0.5h and room temperature for 1.5h. Progress of the reaction was monitored by thin layer chromatography (methanol:dichloromethane 1:9). The reaction was then cooled to 0°C before the careful addition of saturated aq. NaHCO₃ (15ml). This mixture was allowed to stir for 20mins before addition of ethyl acetate (25ml). Stirring was continued for 15mins before separation of the layers. The organic portion was washed further with brine (2 x 15ml) before drying (Na₂SO₄), filtration and concentration in vacuo yielding an off-white solid. This was slurried in diethylether:hexane (1:1) (20ml), filtered and air-dried yielding resveratrol as a beige solid (0.39g, 92%).

## Claims

1. Process for the preparation of resveratrol, comprising the steps of
a) reaction of a compound according to formula I 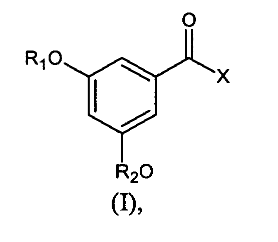 with a compound according to formula II 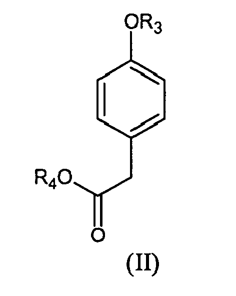 to form a compound according to formula III 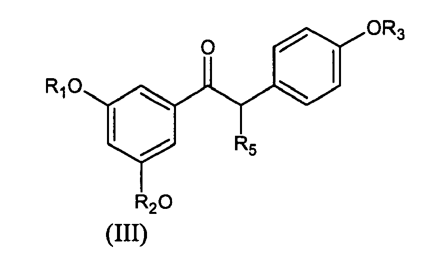
b) conversion of the compound according to formula III to a compound according to formula IV 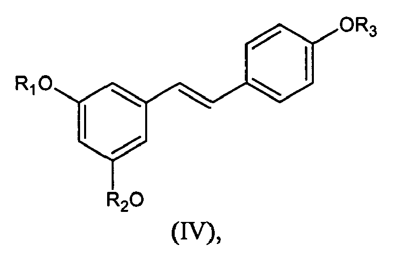 and, if necessary,
c) deprotection of the compound according to formula IV to resveratrol,
wherein R₁, R₂, and R₃ independently from each other represent hydrogen, (C₁-C₄)alkyl group such as methyl, ethyl, n-propyl, iso-propyl, n-butyl, iso-butyl, sec-butyl, tert-butyl; (C₁-C₄)alkoxy(C₁-C₄)alkyl group such as methoxymethyl, methoxyethyl, methoxypropyl, ethoxymethyl, ethoxyethyl, ethoxypropyl; (C₁-C₄)alkoxy(C₁-C₄)alkoxy(C₁-C₄)alkyl group such as methoxyethoxymethyl, methoxyethoxyethyl; allyl, vinyl, silyl, formyl, acyl group such as acetyl, propanoyl, butanoyl or benzoyl; aryl(C₁-C₄)alkyl or substituted aryl(C₁-C₄)alkyl group such as benzyl, phenethyl, diphenylmethyl, p-methoxybenzyl, p-nitrobenzyl, p-methylbenzyl or o-chlorobenzyl; R₄ represents a (C₁-C₁₈)alkyl group such as methyl, ethyl, n-propyl, isopropyl, tert-butyl, decyl, or said groups having an additional substitution such as methoxyethyl, a (C₁-C₁₀)aryl group such as phenyl optionally with additional substituents on the aromatic residue such as 4-methylphenyl, 4-methoxyphenyl, or an aryl(C₁-C₄)alkyl group such as benzyl or phenethyl; R₅ represents hydrogen or a residue COOR₄; and X is a leaving group or hydroxyl group.

2. Process according to claim 1, wherein as an intermediate reaction product in step b) a compound of formula V 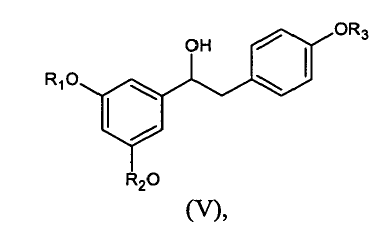 wherein R₁, R₂ and R₃ and R₅ are defined as in claim 1, is formed.

3. Process according to claim 1 or 2, wherein X is selected from fluoride, chloride, bromide, iodide, methoxy, ethoxy, n-propoxy, isopropoxy, n-butanoxy, sec-butoxy, tert-butoxy, phenoxy, 4-chlorophenoxy, 4-bromophenoxy, 4-nitrophenoxy, imidazolyl, trifluorocarboxy, acetoxy, tosylate and mesylate.

4. Process according to claim 1 or 2, wherein X represents OH which is converted *in situ* to fluoride, chloride, bromide, iodide, methoxy, ethoxy, n-propoxy, isopropoxy, n-butanoxy, sec-butoxy, tert-butoxy, phenoxy, 4-chlorophenoxy, 4-bromophenoxy, 4-nitrophenoxy, imidazolyl, trifluorocarboxy, tosylate and mesylate.

5. Process according to any one of claims 1 to 4, wherein step a) is conducted in the presence of a base.

6. Process according to claim 5, wherein the base is selected from sodium hydride, potassium hydride, lithium diisopropylamide, lithium hexamethyldisilylamide, butyl lithium, methyl lithium, potassium butoxide, alkaline alkoxides and earth alkaline alkoxides, such as sodium ethoxide, sodium methoxide, magnesium ethoxide; amidines, guanidines, tetramethylammonium hydroxide, tetrabutylammonium hydroxide, combinations of alkaline hydroxides, such as sodium hydroxide and potassium hydroxide, with phase transfer catalysts, and mixtures thereof.

7. Process according to claim 4, wherein the base is selected from sodium hydride, potassium hydride and calcium hydride.

8. Process according to any one of claims 1 to 4, wherein the compound of formula II is used in its enolate form which optionally may be silylated.

9. Process according to any one of claims 1 to 8, wherein R₅ represents a residue COOR₄.

10. Process according to claim 9, wherein the compound according to formula III is decarboxylated so that R₅ represents hydrogen.

11. Process according to any one of claims 2 to 10, wherein the compound according to formula III is reduced to the compound according to formula V by interaction with a reducing agent selected from NaBH₄ or by catalytic hydrogenation over Pd/C.

12. Process according to any one of claims 1 to 10, wherein the compound according to formula V is converted to the compound according to formula IV by using a catalytic amount of iodine in toluene.

13. Process according to any one of claims 1 to 10, wherein the compound according to formula V is converted to the compound according to formula IV by converting the hydroxyl group to be eliminated to the corresponding mesylate moiety and eliminating the mesylate moiety by using a base.

14. Process according to claim 13, wherein the base is selected from trimethylamine, triethylamine, ethyldiisopropylamine and tributylamine.

15. Process according to any one of claims 1 to 10, wherein the compound of formula IV is deprotected to resveratrol by action of BBr₃ in toluene in the temperature range of -5 to 5 °C.

16. A compound of formula (III) where the residues R₁, R₂, R₃, and R₅ are as defined in Claim 1.

17. The compound 3-(3,5-dimethoxyphenyl)-2-(4-methoxyphenyl)-3-oxopropionic acid methyl ester.

18. The compound 3-(3,5-dimethoxyphenyl)-2-(4-methoxyphenyl)-3-oxopropionic acid ethyl ester.

19. The compound 1-(3,5-dimethoxyphenyl)-2-(4-methoxyphenyl)ethanone.

20. A compound of formula (V) where the residues R₁, R₂, R₃ and as defined in Claim 1.

21. The compound 1-(3,5-dimethoxyphenyl)-2-(4-methoxyphenyl)ethanol.
